# EUROPEAN PATENT APPLICATION

(11) **EP 1 217 081 A2**
(43) Date of publication of application: **26.06.2002**
(21) Application number: 01310731.3
(22) Date of filing: 20.12.2001
(51) Int. Cl.: C12Q 1/68

(54) **Methods and reagents for detecting increased risk of developing an inflammatory disorder**

(30) Priority: 22.12.2000 US 258034 P
(71) Applicant: Pfizer Products Inc., Groton, Connecticut 06340 (US)
(72) Inventor: Hall, Stephanie Kathryn, Groton, Connecticut 06340 (US); Milos, Patrice Marie, Groton, Connecticut 06340 (US); Seymour, Albert Barnes, Groton, Connecticut 06340 (US)
(74) Representative: Hayles, James Richard

(57) **Abstract**

The present invention relates to methods for reliably detecting an increased risk of developing an inflammatory disorder in a mammalian patient (*e.g.*, a human being) by detecting at least one copy of an IL-1β gene haplotype in the patient comprising cytosine nucleotides at positions -31 and +3953. Also provided are kits for performing such methods. In addition, methods for detecting patients who require a higher dosage of an agent that reduces the effect of IL-1β are also provided.

## Description

### FIELD OF THE INVENTION

This invention relates to the fields of genetics, genomics, and immunology. More specifically, this invention relates to methods for the detection of an increased risk for developing an inflammatory disorder.

### BACKGROUND OF THE INVENTION

Inflammation has recently been described as a complex protective physiologic response elicited by various stimuli such as infectious agent, localized tissue injury, or other trauma (Gallin *et al.,* Inflammation: Basic Principles and Clinical Correlates, Raven Press, New York (1988)). The inflammatory response involves numerous mediators and various immune cells. The migration of white blood cells from the blood stream through the endothelial cell wall to sites of injury, infection, or immunological reaction is recognized as a characteristic feature and a critical step of the inflammatory response (Issekutz *et al., Immunol*. 88:569-576 (1996)). The accumulation of leukocytes into inflamed tissues occurs as a consequence of endothelial cell activation and leukocyte migration to the inflammatory foci.

Numerous human inflammatory disorders and diseases result from abnormal inflammatory responses including, without limitation, coronary artery disease, osteoporosis, nephropathy in diabetes mellitus, alopecia areata, Graves' disease, systemic lupus erythematosus, lichen sclerosis, ulcerative colitis, periodontal disease, juvenile chronic arthritis, chronic iridocyclitis, psoriases, insulin dependent diabetes, diabetic complications, diabetic retinopathy, atherosclerosis, Crohn's disease, rheumatoid arthritis, osteoarthritis, congestive heart failure, neurodegenerative diseases, and any other disease with an inflammatory component.

Interleukin-1 (IL-1) is a cytokine comprising both IL-1α and IL-1β that is able to initiation a wide variety of proinflamatory activities (Bevilacqua *et al*., *Science* 243:1160-1164 (1989); Guan *et al., J. Bio. Chem*. 273:28670-28676 (1998); Flannery *et al., Matrix Bio.* 18:225-236 (1999)). The gene cluster encoding IL-1, which in humans lies on chromosome 2q, has been reported to be the site of polymorphisms (see, for example, EI-Omar *et al*., *Nature* 404:398-402 (2000)). Piciot *et al.* reported that a polymorphism in the IL-1β gene allegedly correlates with increased IL-1β secretion *in vitro* (Piciot *et al*., *Eur*. *J*. *Clin*. *Invest.* 22:396-402 (1992)). Several studies have analyzed whether or not these polymorphisms in the IL-1 gene are associated with inflammatory disorders.

In certain studies, IL-1 gene polymorphisms have been described as being associated with inflammatory disease. For example, PCT publication WO98/54359 (PCT Application No. PCT/GB98/01481), and counterpart U.S. Patent No. 6,268,142, describe a method involving the detection of at least one allele in a IL-1 haplotype that allegedly allows detection of a propensity to develop an inflammatory disorder. The alleles described in WO98/54359 and U.S. Patent No. 6,268,142 include one IL-1β allele having a polymorphism at position +3953, and another IL-1β allele having a polymorphism at position -511. Zheng *et al.* describe the occurrence of a polymorphism in exon 5 of IL-1β, and conclude that this polymorphism does not confer susceptibility to the development of multiple myeloma (Zheng *et al*., *Br*. *J*. *Haematology* 109:39-45 (2000)). Takamatsu *et al*. describe an IL-1β allele marked by polymorphisms at -511 and +3953 which is allegedly associated with the development of alcoholic cirrhosis in Japanese patients (Takamatsu *et al*., *Am. J*. *Gastroenterol.* 95:1305-1311 (2000)). Huang et *al*. describe the presence of a IL-1β gene polymorphism in exon 5 and allegedly demonstrate that myasthenia gravis patients with a polymorphism in the IL-1 receptor antagonist (IL-1 Ra) gene have a statistically higher percentage of this IL-1β exon 5 polymorphism (Huang *et al*., *Journal of Neuroimmunol.* 81:76-81 (1998)). EI-Omar et al. describe an IL-1β haplotype marked by polymorphisms at -511 and -31 which is allegedly associated with increased risk of gastric cancer (El Omar et *al*., *Nature* 404:398-402 (2000)).

However, Santtila *et al.* teach that these IL-1β gene polymorphisms at positions -511 and +3953 are not major regulators of the *in vitro* IL-1β production (Santtila *et al., Scand*. *J*. *Immunol.* 47:195-198 (1998)). Other studies also state that there is no correlation between IL-1 gene polymorphisms and inflammatory disease. For example, Hacker *et al.* studied patients with ulcerative colitis or Crohn's disease and concluded that there was no difference in the patient with these inflammatory bowel diseases as compared to healthy controls with regard to the allele and genotype frequencies of IL-1β polymorphism (Hacker *et al*., *Eur*. *J*. *of Clin*. *Invest.* 28:214-219 (1998)). Cookson *et al*. studied patients with type I autoimmune hepatitis (AIH) and concluded that there were no significant differences in the distributions of the IL-1β alleles, genotypes, or haplotypes in AIH patients and healthy controls (Cookson *et al*., *Hepatology* 30:851-856 (1999)).

Given the discrepancies in the previous studies of the association of polymorphisms in the human IL-1 gene cluster with inflammatory disorders, there remains a need for a reliable method for detecting an increased risk for developing an inflammatory disorder in patients. Such an early detection method could aid in decreasing or even preventing the damaging effects of an abnormal or excessive inflammatory response on the body.

All of the documents cited herein, including the foregoing, are incorporated by reference herein in their entireties.

### SUMMARY OF THE INVENTION

This invention is based on the observation that when at least one copy of a haplotype of the human IL-1β gene comprising a cytosine nucleotide at position -31 and a cytosine nucleotide at position +3953 is present in the genome of a mammalian patient (e.g., a human being), that patient's cells secrete higher amounts of IL-1β in response to suboptimal stimulus. Given the role of IL-1 (of which IL-1β is a component) as a primary mediator in the pathogenesis of inflammatory diseases and disorders, excessive secretion of IL-1β protein by a patient's cells in response to suboptimal stimulus is associated with an increased risk of developing an inflammatory disorder by the patient. Accordingly, this invention provides a reliable method for detecting an increased risk for developing an inflammatory disorder in a patient. Such an increased risk can be detected by detecting the presence of at least one copy of an IL-1β gene haplotype defined by the presence of cytosine nucleotides at positions -31 and +3953 in that patient.

Accordingly, in a first aspect, the invention provides a method for detecting an increased risk for developing an inflammatory disorder in a mammal, comprising detecting the presence of at least one copy of an IL-1β gene haplotype, the haplotype comprising cytosine nucleotides at positions -31 and +3953 in the mammal. The presence of at least one copy of the IL-1β gene haplotype comprising cytosine nucleotides at positions -31 and +3953 indicates that the mammal has an increased risk for developing an inflammatory disorder. Preferably, the method detects an increased risk for developing an inflammatory disorder in a human being. Preferably, the mammal has two copies of the IL-1β gene haplotype comprising cytosine nucleotides at positions -31 and +3953 in the mammal.

In various embodiments of the first aspect of the invention, the IL-1β gene haplotype further comprises a thymidine nucleotide at position -511. In certain embodiments, the inflammatory disorder is coronary artery disease, osteoporosis, nephropathy in diabetes mellitus, alopecia areata, Graves' disease, systemic lupus erythematosus, lichen sclerosis, ulcerative colitis, periodontal disease, juvenile chronic arthritis, chronic iridocyclitis, psoriases, insulin dependent diabetes, diabetic complications, diabetic retinopathy, atherosclerosis, Crohn's disease, rheumatoid arthritis, osteoarthritis, congestive heart failure, or a neurodegenerative disease.

In a second aspect, the invention provides a method for detecting a mammalian patient who requires an increased dosage of an agent that reduces the effect of IL-1β. This method comprises detecting the presence of at least one copy of an IL-1β gene haplotype comprising cytosine nucleotides at positions -31 and +3953 in the patient. The presence of at least one copy of the IL-1β gene haplotype comprising cytosine nucleotides at positions -31 and +3953 indicates that the patient requires a dosage of the agent to reduce the effect of IL-1β in the patient that is higher than the dosage of the agent required to reduce the effect of IL-1β in a second mammal who does not have at least one copy of the IL-1β haplotype comprising cytosine nucleotides at positions -31 and +3953.

In preferred embodiments of the second aspect, the mammalian patient having at least one copy of the IL-1β gene haplotype comprising cytosine nucleotides at positions -31 and +3953 has two copies of the IL-1β gene haplotype. Preferably, the mammalian patient having at least one copy of the IL-1β gene haplotype is a human being. Preferably, the mammalian patient and the second mammal are of the same species.

In various embodiments of the second aspect of the invention, the IL-1β gene haplotype further comprises a thymidine nucleotide at position -511. In certain embodiments, the inflammatory disorder is coronary artery disease, osteoporosis, nephropathy in diabetes mellitus, alopecia areata, Graves' disease, systemic lupus erythematosus, lichen sclerosis, ulcerative colitis, periodontal disease, juvenile chronic arthritis, chronic iridocyclitis, psoriases, insulin dependent diabetes, diabetic complications, diabetic retinopathy, atherosclerosis, Crohn's disease, rheumatoid arthritis, osteoarthritis, congestive heart failure, or a neurodegenerative disease.

In a third aspect, the invention provides kits for detecting an increased risk of developing an inflammatory disorder in a mammal. In a preferred embodiment, the kits comprise a first set of PCR primers for amplifying a first DNA sequence comprising the nucleotide at position -31 of an IL-1β gene; a second set of PCR primers for amplifying a second DNA sequence comprising the nucleotide at position +3953 of the IL-1β gene; and instructions for determining the presence of at least one copy of a IL-1β gene haplotype comprising cytosine nucleotides at positions -31 and +3953 in the mammal based on the size of the PCR products. Preferably, the mammal is a human being.

In certain embodiments of the third aspect of the invention, the kit further comprises a third set of primers for amplifying a third DNA sequence comprising the nucleotide at position -511 of the IL-1β gene and instructions for determining the presence of at least one IL-1β gene haplotype comprising cytosine nucleotides at positions -31 and +3953 and a thymidine nucleotide at position -511 in the mammal based on the size of the PCR products.

In certain embodiments, the inflammatory disorder is is coronary artery disease, osteoporosis, nephropathy in diabetes mellitus, alopecia areata, Graves' disease, systemic lupus erythematosus, lichen sclerosis, ulcerative colitis, periodontal disease, juvenile chronic arthritis, chronic iridocyclitis, psoriases, insulin dependent diabetes, diabetic complications, diabetic retinopathy, atherosclerosis, Crohn's disease, rheumatoid arthritis, osteoarthritis, congestive heart failure, or a neurodegenerative disease.

### BRIEF DESCRIPTION OF THE DRAWING

The foregoing and other objects of the present invention, the various features thereof, as well as the invention itself may be more fully understood from the following description, when read together with the accompanying drawing in which:

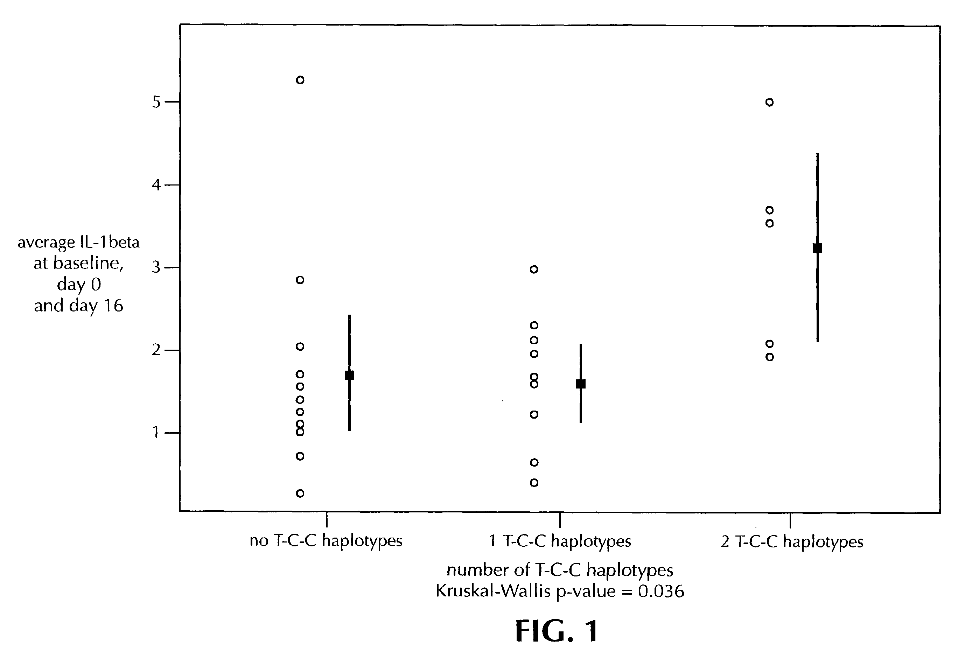

Figure 1 is a schematic representation of a scatter plot from individuals with the indicated IL-1β gene haplotype (on the X axis) showing the average combined IL-1β protein secretion (ng/ml) following lipopolysaccharide (LPS) stimulation only from three independent assays performed three different time points (baseline, day 0, and day 16), as quantitated using an enzyme-linked immunosorbent assay (ELISA). The closed squares with the standard deviation bar indicates the average of all the individuals in the indicated haplotype. Individuals having two IL-1β haplotypes comprising a T at -511, a C at -31, and a C at +3953 show a significantly greater IL-1β secretion in response to stimulation with LPS alone as compared to those individuals with no "TCC" haplotype or only one "TCC" haplotype.

### DETAILED DESCRIPTION OF THE INVENTION

Those skilled in the art will fully understand the terms used herein to describe the present invention; nonetheless, the following terms used herein, unless otherwise provided herein, are as described below.

By "agent that reduces the effect of IL-1β" is meant any chemical that can target the IL-1β pathway and reduce the inflammation caused by IL-1β. Agents of this definition include, without limitation, interleukin-1 receptor antagonist, neutralizing antibodies specific for IL-1β or the IL-1 receptor, Anakinra (produced by Amgen, Inc., Thousand Oaks, CA), and any other small molecules that bind to either the IL-1 receptor or to IL-1β and prevent IL-1β from binding to and activating the IL-1 receptor. The determination of the dosage of such an agent for a mammal who does not have at least one copy of an IL-1β gene haplotype comprising cytosine nucleotides at positions -31 and +3953 is within the skill of the ordinary health care professional (e.g., physician or pharmacist) and will depend upon the particular agent being used.

By "allele" is meant one of at least two alternative forms of a genetic locus, where a single allele for each genetic locus is inherited separately from each parent. If an individual has the same haplotype from each parent for the same gene, that individual is "homozygous" for that gene. If an individual has different haplotypes from each parent for the same gene, that individual is heterozygous for that gene.

By "genotype" is meant the sum total of the elements associated with a single genetic locus. Thus, genotyping a patient allows the determination of whether the patient has a copy (or two copies) of an IL-1β gene haplotype of the invention.

By "haplotype" is meant the particular combination of closely linked alleles or markers found in the same region of a chromosome (e.g., found in the same gene). For example, an IL-1β gene haplotype of the invention is one that has the following markers: a cytosine nucleotide ("C") at position -31 and a cytosine nucleotide at position +3953.

By "increased risk for developing an inflammatory disorder" is meant an individual determined to have an increased risk for developing an inflammatory disorder according to the methods of the invention. Such an individual has a greater predisposition for developing the disorder than if the individual were determined to not have an increased risk for developing an inflammatory disorder according to the methods of the invention. Preferably, the individual determined to have an increased risk for developing an inflammatory disorder according to the methods of the invention has a greater predisposition for developing the disorder as compared to an individual subjected to similar environmental conditions, but who is determined not to have an increased risk of an inflammatory disorder. The increased risk is preferably at least about 10% higher in an individual determined according to the methods of the invention to have an increased risk for developing an inflammatory disorder as compared to an individual determined not to have an increased risk for developing an inflammatory disorder. More preferably, the increased risk is at least about 25% higher; still more preferably, the increased risk is at least about 50% higher; even more preferably, the increased risk is at least about 75% higher; even more preferably, the increased risk is at least about 100% higher (*i.e.*, about 2 fold higher); even more preferably, the increased risk is at least about 200% higher (*i.e.*, about 4 fold higher); and most preferably, the increased risk for developing an inflammatory disorder in an individual determined to have an increased risk for developing an inflammatory disorder is at least about 400% higher (*i.e.*, 8 fold higher) as compared to an individual determined not to have a appreciable increased risk for developing an inflammatory disorder. Preferably, the compared individuals are matched for species, gender, age, and/or exposure to similar environmental conditions. Methods for determining whether or not an individual has developed an immune disorder are known to the ordinarily skilled health care professional, such as a nurse or a physician. Such methods include, without limitation, detection of an increase in white blood cell count, detection of redness, detection of swelling (as measured, for example, by calipers), and/or detection of fever.

By "inflammatory disorder" is meant any disease or disorder characterized by an abnormal and/or excessive inflammatory response. Inflammatory disorders include, without limitation, coronary artery disease, rheumatoid arthritis, osteoporosis, nephropathy in diabetes mellitus, alopecia areata, Graves' disease, systemic lupus erythramatosus, lichen sclerosis, ulcerative colitis, periodontal disease, juvenile chronic arthritis, chronic iridocyclitis, psoriases, insulin dependent diabetes, diabetic complications, diabetic retinopathy, atherosclerosis, Crohn's disease, osteoarthritis, congestive heart failure, neurodegenerative diseases, and any other diseases with an inflammatory component.

By "linkage disequilibrium" is meant the state in which the frequencies of the different haplotypes present do not conform to a random distribution as predicted by the individual frequencies of the various alleles. That is, linkage equilibrium means that two markers (e.g., a C at position -31 in the IL-1β gene and a C at position +3953 in the IL-1β gene) are inherited dependently of each other, and not independently.

By "marker" is meant a difference a nucleotide (*e.g.*, C instead of T) at a specific position (*e.g.*, at position -31) in the DNA sequence of a gene (*e.g.*, IL-1β gene) among individuals in a population. Thus, one allele of the human IL-1β gene has a cytosine nucleotide at position -31 and another allele of the human IL-1β gene has a thymidine nucleotide at position -31. By convention of usage in the genomics art, the presence of a cytosine nucleotide at a position in a gene is called allele 1 of that gene and the presence of a thymidine nucleotide at a position in a gene is called allele 2 of that gene (EI-Omar *et al*., *Nature* 404: 398-402 (2000)).

The published patent and scientific literature referred to herein establishes knowledge that is available to those with skill in the art. The U.S. patents, allowed applications, published foreign patent applications, and references, including GenBank database sequences, that are cited herein are hereby incorporated by reference to the same extent as if each was specifically and individually indicated to be incorporated by reference. Any inconsistency between these publications and the present disclosure shall be resolved in favor of the present disclosure.

The present invention provides reliable methods for detecting a predisposition to develop an inflammatory disease in a mammalian patient, particularly a human being. The methods of the invention are useful as analytical tools and as therapeutic tools. The invention also provides methods which may be manipulated and fine-tuned by those skilled in the art based on the present description to fit the condition(s) to be detected. Standard reference works setting forth the general principles of the genetic and molecular biology technology described herein include Ott and Hoh, "Statistical Approaches to Genetic Mapping," *Am. J. Hum.* Genet. 67:289-294 (2000); Zubay G., Genetics The Benjamin/Cummings Publishing Co., Inc., Menlo Park, CA (1987); Ausubel *et al*., Current Protocols in Molecular Biology, John Wiley & Sons, New York, NY (1999); Sambrook *et al*., Molecular Cloning: A Laboratory Manual, 2d Ed., Cold Spring Harbor Laboratory Press, Plainview, NY (1989); Kaufman *et al*. (Eds.), Handbook of Molecular and Cellular Methods in Biology and Medicine, CRC Press, Boca Raton, LA (1995); and McPherson, Ed., Directed Mutagenesis: A Practical Approach, IRL Press, Oxford (1991). Standard reference works setting for the general principles of immunology and inflammation include Gallin *et al*., Inflammation: Basic Principles and Clinical Correlates, Raven Press, New York (1988); Kuby, J., Immunology, 3^{rd} ed., W.H. Freeman, New York (1997); Coligan *et al*. (Eds.), Current Protocols in in Immunology, John Wiley & Sons, New York (1991); and Hurley, J.V., Acute Inflammation, 2^{nd} ed., Churchill Livingstone, New York (1983).

The present invention is based on the observation that the presence of a specific haplotype of the IL-1β gene in the genome of a patient is a reliable indicator for that patient having an increased risk for developing an inflammatory disorder. The inventors have discovered at least two distinct alleles in the IL-1β gene which are in linkage disequilibrium.

To understand linkage disequilibrium, it is useful to understand linkage equilibrium, which is the natural state of alleles assuming random mating. In the simplest example, with random mating, alleles at any genetic locus will acquire random association into genotypes of the following specific frequencies: Sperm contains allele A or a for gene X; Egg contains allele A or a for the same gene X; and, accordingly, Offspring will have alleles AA 25% of time, alleles Aa 50% of the time, and alleles aa 25% of the time.

In a more complicated example, for two genes (*e.g.*, gene A at locus 1 with alleles A1 and A2 and frequencies of occurrence denoted p1 and p2 and gene B at locus 2 with alleles B1 and B2 and frequencies denoted as q1 and q2), the frequency at which both are inherited together on the same chromosome is as follows:
allele A1 with allele B1 = p1q1
allele A1 with allele B2=p1q2
allele A2 with allele B1=p2q1
allele A2 with allele B2= p2q2
(Note that since there are only two alleles of each of genes A and B, p1 + p2 =1 and q1 + q2 = 1.) Thus, the frequency of observing these two genes inherited together as a haplotype is the product of their allele frequency.

Using the IL-1β gene markers as an example, the allele frequencies for the - 511 (C or T) marker are, for the purposes of this example, 37% T and 63% C. The allele frequencies for the -31 (T or C), for the purposes of this example, is 40%C and 60% T. Therefore, the expected frequency of observing the T at -511 and the C at - 31 would be (37%) X (40%) = 14.8%. If the observed frequency of inheriting the T at -511 and the C at -31 together reaches a statistically significant value higher or lower than the expected 14.8%, then these two markers are said to be in linkage disequilibrium (where statistical significance is as described, e.g., in Joseph Terwilliger and Jurg Ott, Handbook of Human Genetic Linkage, The Johns Hopkins University Press (1994)).

Therefore, the formula for calculating disequilibrium is: D_{AB} = P_{AB} - (P_{A} X P_{B}), where "D_{AB}" stands for linkage disequilibrium of allele A (*e.g.,* nucleotide T) at locus 1 (*e.g.*, -511 on the IL-1 gene) and allele B (*e.g.*, nucleotide C) at locus 2 (e.g., -31on the IL-1β gene); where "P_{AB}" stands for the frequency of A at locus 1 with B at locus 2; where "P_{A}" stands for the frequency of allele A at locus 1; and where "P_{B}" stands for the frequency of allele B at locus 2. Where two markers (*e.g.*, alleles A at locus 1 and allele B at locus 2) are not in linkage disequilibrium, the value for D_{AB} should be 0.

The linkage disequilibrium information can be used to estimate haplotypes through the use of a statistical algorithm called the Expectation Maximization (EM) algorithm, which is used by the statistical software package called Estimating Haplotype-frequencies (EH) (ftp://linkage.rockefeller.edu/software/eh) designed by Jurg Ott (explained in Joseph Terwilliger and Jurg Ott, Handbook of Human Genetic Linkage, The Johns Hopkins University Press (1994)). Haplotype frequencies for pairs of alleles are estimated, for example, using the EH software program. One estimate of linkage disequilibrium, D' = D_{AB} /Dₘₐₓ (where Dₘₐₓ= min (p1q1, p2q2, where p and q are allele frequencies for the two respective markers in question), and χ² values are calculated for pairs of the most common alleles at each locus using the LINKDOS software program (distributed with GENEPOP, ftp://ftp.cefe.cnrs-mop.fr/pub/PC/MSDOS/GENEPOP/). Odds ratios with Cornfield 95% confidence intervals and logistic regression models controlling for the effects of possible confounders can be computed using STATA version 5.0 software (STATA Press). D' was calculated according to formulas which can be found in "Genetic Data Analysis II: Methods for Discrete Population Genetic Data", Bruce Weir, Sinauer Associates, Inc., Sunderland, MA 1996.

The two (or three) markers identified herein as being in linkage disequilibrium define the specific allele of the IL-1β gene, the presence of which is a reliable indicator for increased risk of developing an inflammatory disorder.

The inventors have found that two markers, namely cytosine at position -31 and cytosine at position +3953, are in linkage disequilibrium in the human IL-1β gene, where the +1 position indicates the start site of transcription. Position -31 is in the regulatory portion of the IL-1β gene upstream (*i.e.*, 5') of the start site of transcription, whereas position +3953 is in exon 5 of the IL-1β gene. Note that, depending upon the method used for numbering the start site of transcription, position +3953 is sometimes numbered as +3954 (see Kornman *et al*., *J. Periodontal Res.* 34:353-357 (1999)). Regardless, this site is recognized by the Taql restriction endonuclease, which specifically recognizes the sequence 5'...TCGA...3'. When the cytosine nucleotide appears at position +3953 in the IL-1β gene, the Taq I recognition site is present.

Moreover, as described in detail in Example I below, when at least one copy, or preferably two copies, of a haplotype of IL-1β comprising a cytosine nucleotide at position -31 and a cytosine nucleotide at position +3953 is present in the genome of a patient, that patient's cells secrete higher amounts of IL-1β in response to suboptimal stimulus (as compared to the amount of IL-1β secreted by cells of a patient whose genome does not have at least one copy of a IL-1β haplotype comprising C nucleotides at positions -31 and +3953). Since interleukin-1 (IL-1) is a primary mediator in the pathogenesis of inflammatory diseases and disorders, and since IL-1β is a component of IL-1, excessive secretion of IL-1β protein by a patient's cells in response to suboptimal stimulus is associated with an increased risk of developing an inflammatory disorder by the patient.

Accordingly, the invention provides a method for detecting an increased risk for developing an inflammatory disorder in a mammalian patient. This method comprises detecting the presence of at least one copy of an IL-1β gene haplotype comprising cytosine nucleotides at positions -31 and +3953 in the patient, where the presence of at least one copy of the IL-1β gene haplotype comprising cytosine nucleotides at positions -31 and +3953 indicates that the patient has an increased risk for developing an inflammatory disorder. Preferably, the patient is a human being. Preferably, the patient who has at least one copy of an IL-1β gene haplotype comprising cytosine nucleotides at positions -31 and +3953 has two copies of an IL-1β gene haplotype comprising cytosine nucleotides at positions -31 and +3953.

While the patient of the invention is preferably a human being, the methods and kits of the invention may also be employed with other mammals such as, for example, companion animals (*e.g.,* cats, dogs, horses, elephants), livestock (*e.g.,* cattle, sheep, goats, pigs), and laboratory animals (*e.g.*, mice, rats, non-human primates).

This invention further relates to a third polymorphism that occurs at position -511 in the IL-1β gene haplotype of the invention. Thus, when cytosine nucleotides are found to occur at positions -31 and +3953 in a patient's IL-1β gene, the patient has an even more pronounced increased risk for developing an inflammatory disorder when the nucleotide found at position -511 in that IL-1β allele is a thymidine. Accordingly, the IL-1β gene haplotype may further comprises a thymidine nucleotide at position -511.

Moreover, it is well known that inflammatory cytokines, such as IL-1β, are involved with diseases (including, without limitation, rheumatoid arthritis and psoriasis), and animal models have shown that by reducing the effects of IL-1β, through recombinant forms of its natural antagonist IL-1RA, the disease effects of IL-1β can be reduced. Currently, there are no therapies on the market that specifically inhibit IL-1; however, Anakinra, produced by Amgen, Inc. (Thousand Oaks, CA), a recombinant form of the natural receptor antagonist IL-1 RA, has been reported to produce improvements in the signs and symptoms of rheumatoid arthritis and a reduction in the progression of joint destruction in phase II trials.

Thus, therapies specifically blocking the effects of IL-1β could be influenced by the concentration of extracellular IL-1β that differs between individuals being treated. Based upon the present description, once knowledge of a subject's IL-1β haplotype is obtained, such knowledge can be used to determine appropriate dosing of future therapies targeting the IL-1β pathway. In one example, subjects homozygous for the TCC haplotype may require larger doses or prolonged therapy to obtain equivalent pharmacological inhibition and/or clinical response observed in subjects carrying only one or zero copies of this haplotype.

Accordingly, the invention also provides methods for detecting a mammalian patient who requires an increased dosage of an agent that reduces the effect of IL-1β comprising detecting the presence of at least one copy of an IL-1β gene haplotype comprising cytosine nucleotides at positions -31 and +3953 in the patient. The presence of at least one copy of the IL-1β gene haplotype comprising cytosine nucleotides at positions -31 and +3953 indicates that the patient requires a dosage of the agent to reduce the effect of IL-1β in the patient that is higher than the dosage of the agent required to reduce the effect of IL-1β in a second mammal who does not have at least one copy of the IL-1β haplotype comprising cytosine nucleotides at positions -31 and +3953.

Preferably, the patient having at least one copy of the IL-1β gene haplotype comprising cytosine nucleotides at positions -31 and +3953 has two copies of the IL-1β gene haplotype. Preferably, the patient having at least one copy of the IL-1β gene haplotype is a human being. Preferably, the mammalian patient and the second mammal are of the same species, age, and/or are exposure to similar environmental conditions.

In certain embodiments, the IL-1β gene haplotype comprises cytosine nucleotides at positions -31 and +3953 and a thymidine nucleotide at position -511.

The inflammatory disorder of the methods of the invention may be coronary artery disease, osteoporosis, nephropathy in diabetes mellitus, alopecia areata, Graves' disease, systemic lupus erythematosus, lichen sclerosis, ulcerative colitis, periodontal disease, juvenile chronic arthritis, chronic iridocyclitis, psoriases, insulin dependent diabetes, diabetic complications, diabetic retinopathy, atherosclerosis, Crohn's disease, rheumatoid arthritis, osteoarthritis, congestive heart failure, or a neurodegenerative disease.

This invention is also directed to kits for detecting increased risk for developing an inflammatory disorder in a mammalian patient, such as a human being. These kits allow for the rapid detection of the presence of an IL-1β gene haplotype of the invention (e.g., comprising a C at position -31 and a C at position +3953). Accordingly, the invention also provides kits for detecting an increased risk of developing an inflammatory disorder in a patient comprising a means for detecting the presence of at least one IL-1β gene haplotype comprising cytosine nucleotides at positions -31 and +3953 in the patient. Preferably, the IL-1β gene haplotype comprises cytosine nucleotides at positions -31 and +3953 and a thymidine nucleotide at position -511.

Any means for detecting the presence of cytosine nucleotides at positions - 31 and +3953 can be used in the kits of the invention. For example, the means may be two sets of polymerase chain reaction (PCR) primers (one set for the -31 position and another for the +3953 position) and reagents for performing PCR to determine the genotype of the patient being tested.

In one non-limiting example, the kit may have PCR means for detecting a C at position -31 and a C at position +3953 in an IL-1β gene, where the presence or absence of these nucleotides would result in a PCR product of different lengths. Thus, if the patient has the IL-1β haplotype of the invention (i.e., C nucleotides at positions -31 and +3953) and so has an increased risk for developing an inflammatory disease, the PCR amplification, using the means of the kit, would result in two PCR product, one 800 basepairs (bp) in length and the other of 400 bp in length. The presence of an IL-1β haplotype not having a C nucleotide at position -31 and +3953 results in two PCR products, one 1.2 kB in length and another 2.0 kB in length. Resolution of the PCR products by agarose gel electrophoresis identifies a patient having at least one copy of the IL-1β haplotype of the invention (four distinct bands at 400 bp, 800 bp, 1.2 kB, and 2.0 kB), a patient having two copies of the IL-1β haplotype of the invention (two distinct bands at 400 bp and 800 bp), and a patient having no copies of the IL-1β haplotype of the invention (and thus at no increased risk for developing an inflammatory disorder) (two distinct bands at 1.2 kB and 2.0 kB). The kit can include instructions describing and explaining the determination of the presence or absence of an IL-1β haplotype of the invention based on occurrence of these distinct bands.

In another non-limiting example, the kit contains PCR primers and amplification instructions as described below in Example I. The kit may additionally include the following restriction enzymes and their digestion buffers, Ava I, Nla III, and Taq I, and instructions for digesting the PCR product from the C-511T sample with Ava I, the C+3953T sample with Taq I, and the T-31C sample with Nla III. The kit may further include directions for resolving the Taq I digested C+3953T samples and the Ava I digested C-511T samples on a 2.5% agarose gel, and the Nla III digested T-31C samples on a 3% agarose gel. The kits may further include instructions for visually examining the resolved bands from the digests where the expected allele fragment are as follows: C-511T sample: the C allele if two bands at 191 bp and 113 bp; the T allele if a single band at 304 bp; T-31C sample: the T allele one band at 117 bp; the C allele if two bands at 100 bp and 17 bp; and C+3953T sample: the C allele if two bands at 135 bp and 114 bp.

Another method for detecting the presence of an IL-1β haplotype of the invention in a patient is restriction fragment length polymorphism or RFLP (described, *e.g.*, in Ausubel *et al*., Current Protocols in Molecular Biology, John Wiley & Sons, New York, NY (1999)). Thus, the kits of the invention include any suitable restriction endonucleases (including, for example, the Taq I restriction endonuclease) and an instruction booklet showing the restriction fragment lengths observed in a patient with no copies of the IL-1β haplotype of the invention, a patient with one copy of the IL-1β haplotype of the invention, and a patient with two copies of the IL-1β haplotype of the invention.

Another non-limiting example of a method to detect the presence of an IL-1β haplotype of the invention is by polymerase chain reaction single-strand conformation polymorphism (PCR-SSCP). In one non-limiting embodiment of such a method, the method for determining genotypes of subjects is as follows: cells are obtained from the subjects, the cells' DNA is isolated according to standard methods, and the cells' genotypes are determined using the polymerase chain reaction single-strand conformation polymorphism (PCR-SSCP) and/or the 5' nuclease PCR assays. For PCR-SSCP, 50 ng DNA can be amplified in a GeneAmp PCR System 9700 (PE Applied Biosystems, Mississauga, Ontario, Canada), using primer pairs that will expand the portion of the IL-1β gene containing the polymorphism of the haplotype. PCR amplification can be performed in a volume of 20 µl, containing 10 mM Tris-HCI pH 8.3, 50 mM KCI, 1.5 mM MgCl₂, 200 µM each of dATP, dTTP and dGTP, 100µM dCTP, 1 µCi of |*a*-³²P|dCTP (3,000 Ci mol⁻¹), 80 ng of each primer, and 1 unit of Taq polymerase. The thermocycling conditions are, for example, 94°C for 10 min.; then 5 cycles of 94°C for 30 sec, 65°C for 30 sec and 72°C for 30 sec; then 30 cycles of 94°C for 30 sec, 60°C for 30 sec and 72°C for 30 sec; then 5 cycles of 94°C for 30 sec, 55°C for 30 sec and 72°C for 30 sec. SSCP analysis of the radiolabelled amplification products can then be performed according to standard methods (see, *e.g.,* Law *et al*., *Analytical Biochemistry* 236: 373-375 (1996)).

Yet another way to detect the presence of an IL-1β haplotype of the invention is by the 5' nuclease PCR assay (TaqMan, PE Applied Biosystems). Primers and probes can be designed using, for example, the Primer Express software (PE Applied Biosystems). Probes for the T or C allele can be 5'-labelled with either FAM (6-carboxyfluoresceine) or V1C fluorogenic dyes, and 3'-labeled with TAMRA (6-carboxytetramethylrhodamine) quencher. PCR amplification can be performed in a volume of 25 µl containing 50 ng genomic DNA, I X TaqMan Universal Master Mix (PE Applied Biosystems), 200 nM of each probe, and 900 nM of primers. Amplification conditions can be, for example, 50°C for 2 min, 95°C for 10 min, then 40 cycles of 95°C for 15 sec and 62°C for 1 min, as recommended by the manufacturer. Thermal cycling of optical plates can be performed in the ABI PRISM 7700 Sequence Detection System (PE Applied Biosystems).

Additional methods for detecting the presence of an IL-1β haplotype of the invention include, without limitation, sequence analysis, molecular haplotyping, fluorescence polarization, mass spectrometry, fluorescence resonance energy transfer or FRET, any methodology that reads the product of a primer-extension reaction, and hybridization-based technologies. Such methods are well known to those of skill in the art and are described, e.g., in Ausubel *et al*., Current Protocols in Molecular Biology, John Wiley & Sons, New York, NY (1999) and Sambrook *et al*., Molecular Cloning: A Laboratory Manual, 2d Ed., Cold Spring Harbor Laboratory Press, Plainview, NY (1989).

In another non-limiting example, the presence of an IL-1β haplotype of the invention is shown to be causative for an increased IL-1β protein secretion in response to suboptimal stimulus and, accordingly, an increased risk in a patient for developing an inflammatory disorder. In this non-limiting example, the blood cells from patients described in Example I are immortalized (e.g., by infection with Epstein-Barr Virus (EBV)). Once stable cell lines are established, the levels of IL-1β protein secreted by the cells following no stimulus, stimulus with LPS only, or stimulus with LPS followed by ATP are determined.

Artificial chromosomes may be generated, where the chromosomes comprises the entire IL-1β gene (human IL-1β gene sequence is available, for example, from GenBank at Accession Nos. U26540, M15330, M15534; M15534; M15840; M15840; AF043335). Particularly, at least two artificial chromosomes are generated, one which has an IL-1β gene comprising a C at position -31 and a C at position +3953; and another which has an IL-1β gene comprising a T at position -511, a C at position -31, and a C at position +3953.

The artificial chromosome according to this example may be designed to exist episomally once transfected into a cell, or may be designed to integrate into the genome of the cell to which it is transfected using homologous recombination. Because the transfection of an episomal chromosomal into a cell would introduce a third copy of the IL-1β gene, the homologous recombination method is preferably employed. However, since the presence of the IL-1β haplotype of the invention (*i.e.*, comprising either C at position -31 and C at position +3953 or T at position -511, C at position -31, and C at position +3953) does not affect the production of the IL-1β protein, but rather, affects the secretion of the intracellularly stored protein, it is likely that an effect on IL-1β protein secretion will be observed even with the episomal artificial chromosome.

For generation of an episomal artificial chromosome, for example, if the cells are immortalized by infection with EBV, then an artificial chromosome may be generated on a circular vector, where the vector comprises the IL-1β gene, a selectable marker (e.g., sequence encoding resistant to hygromycin), and the OriP origin of replication for Epstein Barr Virus. Such EBV vectors containing the OriP sequence and sequences encoding hygromycin resistance (*i.e.,* the hisD sequence) are commercially available from, for example, Invitrogen, Carlsbad, CA). The immortalized cell lines are transfected (*e.g.,* by electroporation or calcium phosphate (CaPO₄) transfection) with the artificial chromosome, and selected for an ability to grow in the presence of hygromycin (or any other drug to which the marker has made the transfected cell resistant including, without limitation, for example, neomycin (G418), puromycin, and tetracycline).

The artificial chromosome designed to be integrated into the genome of a cell may be designed, for example, by flanking the IL-1β gene sequence with two sequences that will undergo homologous recombination with other sequences in the IL-1 gene cluster. Preferably, the artificial chromosome will be designed to include two selectable markers such that integration of the chromosome at the correct locus (i.e., such that the chromosome replaces the cell's original IL-1β gene) can be selected for. The selected cells can then either be retained as heterozygotes (i.e., having one copy of the artificial chromosome and one copy of the original IL-1β gene) or be rendered homozygous (*i.e.*, two copies of the artificial chromosome) using standard techniques. Homologous recombination is well known and is described, for example, in Ausubel *et al*., Current Protocols in Molecular Biology, John Wiley & Sons, New York, NY 1999 (particularly supplement 45, sections 9.15.1 through 9.17.3).

Once cell lines comprising the artificial chromosomes are established, their IL-1β protein secretion is measured following no stimulus, stimulation with LPS only, or stimulation with LPS followed by ATP. These measurements are compared to the levels of IL-1β protein secreted by the immortalized cells not transfected with the artificial chromosome.

The presence of an IL-1β haplotype of the invention in a cell is causative for that cell's increased secretion of IL-1β protein in response to suboptimal stimulus (*i.e.*, stimulus with LPS only). Thus, a cell whose genome had no copies of the IL-1β haplotype defined by T(-511), C(-31), and C(+3953), when transfected with the artificial chromosome containing an IL-1β gene with either C at position -31 and C at position +3953 or T at position -511, C at position -31, and C at position +3953, has increased IL-1β secretion in response to LPS only as compared to the original cell which lacked an IL-1β haplotype of the invention. Furthermore, if the cell comprises two copies of an IL-1β gene with either C at position -31 and C at position +3953 or T at position -511, C at position -31, and C at position +3953, that cell has an even more pronounced increase in IL-1β secretion in response to LPS only as compared to the original cell which lacks a "TCC" IL-1β haplotype of the invention.

The following examples are intended to further illustrate certain preferred embodiments of the invention and are not limiting in nature. Those skilled in the art will recognize, or be able to ascertain, using no more than routine experimentation, numerous equivalents to the specific substances and procedures described herein. Such equivalents are considered to be within the scope of this invention, and are covered by the following claims.

### EXAMPLE I

### Presence of an IL-1β Haplotype Correlated With Increased IL-1β Protein Secretion in Response to Suboptimal Stimulus

The genotypes were determined for thirty-one healthy Caucasian human subjects of Northern European descent. To do this, the following primers flanking the indicated IL-1β gene polymorphisms were generated:

C-511T:
Forward PCR primer sequence: 5'-TGGCATTGATCTGGTTCATC-3' (SEQ ID NO: 1)
Reverse PCR primer sequence: 5'-GTTTAGGAATCTTCCCACTT-3' (SEQ ID NO: 2)

T-31C: (PCR1)
Forward PCR primer sequence: 5'-CCAATACTCTTTTCCCCTTTCC-3' (SEQ ID NO:
3)
Reverse PCR primer sequence: 5'-CTTGTGCCTCGAAGAGGTTT-3' (SEQ ID NO:
4)

T-31C: (PCR2)
Forward PCR primer sequence: 5'-CCAATACTCTTTTCCCCTTTCC-3' (SEQ ID NO:
3)
Reverse PCR primer sequence: 5'-TTCTCCCTCGCTGTTTTCA-3' (SEQ ID NO: 5)

C3953T:
Forward PCR primer sequence: 5'-GTTGTCATCAGACTTTGACC-3' (SEQ ID NO: 6)
Reverse PCR primer sequence: 5'-TTCAGTTCATATGGACCAGA-3 (SEQ ID NO:
7).

PCR amplifications were performed using a PTC-225 tetrad model by MJ Research, Inc. (Waltham, MA). Note that there were two reactions for the T-31C polymorphism. The first reaction amplified a region that contained the polymorphism at position -31. The second reaction used a reverse primer that introduces a G in place of the A three nucleotides downstream of the T-31 C polymorphism. These two reactions introduced a NlaIII restriction site that can be used to score the genotype, rather than the naturally occurring Alul restriction site which is a very common restriction endonuclease and thus is difficult to use to interpret the genotypes.

To perform the PCR amplifications, 10 ng genomic DNA was amplified in a 50µl reaction containing 10 mM Tris-CI, pH 8.7, 50 mM KCI, 1.5 mM MgCl₂, 200 µM each of the dNTPs, 0.5 µM each of the PCR primers and 2.5 Units HotStarTaq DNA polymerase (Qiagen, Santa Clarita, CA). Samples were cycled at 96°C for 15 min. followed by 30 cycles of 96°C for 30 sec., 58°C for 45 sec., and 72°C for 1 min. A second PCR reaction is necessary for the T-31C marker since the reverse primer in the reaction introduces an Nla III site which has proven to be more reliable in the RFLP assays than the native Alu I site. PCR products from the initial PCR reaction are diluted 1:800 and 1 µl PCR product used as template in the second PCR reaction using the same conditions described above.

For genotyping, the PCR products were digested with the appropriate restriction enzyme and buffer provided by the manufacturer (New England Biolabs, Beverly, MA). 80-100ng PCR product was digested with restriction enzyme in a 50 µl volume. C-511T samples were digested with Ava I and the T-31C samples digested with Nla III at 37°C for 90 minutes. The C3953T samples were digested with Taq^{α} I at 65°C for 2 hours. The Ava I and Taq I digests were resolved on 2.5% agarose gels in 1X TBE buffer (0.1M Tris, 0.09M Boric acid, 0.001M EDTA). The Nla III digests were resolved by gel electrophoresis on a 3% agarose gel in 1X TBE buffer. Genotypes were scored by visual examination of the size of the PCR products present.

| Expected allele fragments: | | |
|---|---|---|
| C-511T | C allele: 191 bp and 113 bp | T allele: 304 bp |
| T-31C | T allele: 117 bp | C allele: 100 bp and 17 bp |
| C3953T | C allele: 135 bp and 114 bp | T allele: 249 bp |

Table I shows a typical EH output from the EH software package designed by Jurg Ott (explained in Joseph Terwilliger and Jurg Ott, Handbook of Human Genetic Linkage, The Johns Hopkins University Press (1994)).

**Table I**

| IL-1β Statistical Estimation of Haplotype | | | | |
|---|---|---|---|---|
| | | | Haplotype Frequency | |
| | | | Independent | w/Association |
| Allele at -511 | Allele at -31 | Allele at +3953 | | |
| C | C | C | 0.202315 | 0.016667 |
| C | C | T | 0.040463 | 0.000000 |
| C | T | C | 0.325463 | 0.450001 |
| C | T | T | 0.065093 | 0.166666 |
| T | C | C | 0.117130 | 0.366665 |
| T | C | T | 0.023426 | 0.000001 |
| T | T | C | 0.188426 | 0.000000 |
| T | T | T | 0.037685 | 0.000000 |

wherein: X²₄=63.5 (where there were 4 degrees of freedom used in the X² test statistics), P<10⁻¹², N=31.

Note that under the column "Independent" in Table I, the program is calculating the expected haplotype frequencies by taking the product of all three allele (*i.e.*, marker) frequencies.

Allele frequencies for the 3 markers rounded up to the nearest percentage are listed in Table II.

**Table II**

| Allele at -511 | Allele at -31 | Allele at +3953 |
|---|---|---|
| C= 0.59 | C=0.38 | C=0.84 |
| T= 0.41 | T= 0.62 | T=0.16 |

The column marked "w/Association" is the observed frequencies of the three alleles being inherited together and is calculated via the EM algorithm. The difference between the expected (*i.e.*, "Independent" and observed (*i.e.*, "w/Association") frequencies in Table I demonstrates that these markers (*i.e.,* T at -511, C at -31 and C at 3953) are in linkage disequilibrium such that they are not inherited independently. Pairwise calculations of linkage disequilibrium (D') show that allele T at -511 and allele C at -31 have a D' value of .984 (p=0). Allele C at -31 and allele C at 3953 have a D' value of .632 (p=.00607). Those skilled in the art will appreciate that these values confirm that these alleles are not independently inherited.

Note that the EH program tests for the inheritance of these markers under the null hypothesis of independence. Accordingly, the X² value tests how significant the observed is different than the expected.

Following genotype determination, an *ex vivo* blood assay was used to measure the levels of secreted IL-1β protein levels. This assay consisted of a two-step procedure in which a blood sample was first stimulated with LPS to promote IL-1β synthesis, followed by treatment with ATP to promote postranslational processing. The IL-1β protein released extracellularly was measured by ELISA.

To do this assay, blood was collected from patients in heparin-containing vaccutainer tubes (commercially available from, e.g., Becton Dickinson, Franklin Lakes, NJ). These samples could be stored on ice for up to four hours with no adverse effect on assay performance. Seven-five microliters (µl) of blood was placed into an individual well of a 96-well place and diluted with 75 µl of RPMI 1640 containing 20 mM Hepes, pH 7.3 (commercially available from, for example, Gibco-BRL). The diluted blood samples were then incubated for two hours in the presence or absence of lipopolysaccharide (LPS; 100-200 ng/ml; *E. coli* serotype 055:B5) (commercially available from Sigma Chemical Co., St. Louis, MO) at 37°C in a 5% CO₂ environment. After incubation, 6 mM ATP (final concentration) was introduced as a secretion stimulation. Thus, a solution of 100 mM ATP in 20 mM Hepes, pH 7.0 was made, and an appropriate volume of this solution was added to each well to obtain a final concentration of 6 mM ATP.

The supematants of the blood cells was measured by ELISA. To do this, LPS and ATP were added at each timepoint, with IL-1β protein measurements taken before addition of LPS, after addition of LPS, and after addition of LPS/ATP. At day 16, the patients were "washed out" to ensure that the they did not have any active study drug in their systems. From these "washed out' patients, cells were collected as above and IL-1β protein measured following stimulation (e.g., with LPS alone and LPS/ATP). The IL-1β assay was performed in its entirety at each timepoint to give us three independent measures to control for assay variability. The ELISA for IL-1β protein was purchased from R&D Systems (Minneapolis, MN), and used according to manufacturer's specifications. Absolute IL-1β protein levels were calculated based on comparison of assay performance in the presence of known quantities of recombinant IL-1β protein (included with the ELISA kit) (see also *Journal of Immunology* 165: 4615-4623 (2000)).

Substantially all of the cells from all subjects secreted maximal amounts of IL-1β protein following stimulation with both LPS and ATP. Differences in the level of IL-1β protein secretion were observed among cells from individuals with different IL-1β haplotypes at all time points examined (*i.e.,* at baseline, at day 0, and at day 16). Note that the cells were in LPS for about two hours prior to each reading. Accordingly, the measured levels of IL-1β protein secretion in response to stimulation with LPS only at three different timepoints (baseline, day 0, and day 16) were averaged and correlated with respect to the IL-1β haplotypes. As shown in Figure 1, this analysis revealed a significant association between the inheritance of at least one copy, and more so with two copies of a IL-1β gene haplotype defined by T(-511), C(-31), and C(+3953) and increased levels of IL-1β protein that were secreted in response to suboptimal stimulation with LPS alone (p=0.036).

### EXAMPLE II

### Presence of an IL-1β Haplotype Correlated With An Increased Occurrence of Psoriasis

In another experiment, the genotypes of Caucasian human subjects with psoriasis (and healthy control subjects) was determined as substantially described above in Example I. The results demonstrate that the patients with psoriasis have at least one copy of an IL-1β gene haplotype comprising a cysteine nucleotide at position -31 and a cysteine nucleotide at position +3953 as compared to control subjects (*i.e*., with no psoriasis). Thus, those skilled in the art will, once again, appreciate the usefulness of the methods of the present invention, e.g., in detecting an increased risk in developing an inflammatory disease, condition or disorder.

### EQUIVALENTS

As will be apparent to those skilled in the art to which the invention pertains, the present invention may be embodied in forms other than those specifically disclosed above without departing from the spirit or essential characteristics of the invention. The particular embodiments of the invention described above, are, therefore, to be considered as illustrative and not restrictive. The scope of the invention is as set forth in the appended claims rather than being limited to the examples contained in the foregoing description.

## Claims

1. A method for detecting an increased risk for developing an inflammatory disorder in a mammal, comprising detecting, in a sample comprising DNA taken from said mammal, the presence of at least one copy of an IL-1β gene haplotype comprising cytosine nucleotides at positions -31 and +3953 in said mammal, wherein the presence of said at least one copy of the IL-1β gene haplotype indicates that said mammal has an increased risk for developing said inflammatory disorder.

2. The method as defined in claim **1** wherein said mammal has two copies of said IL-1β gene haplotype.

3. The method as defined in claim **1** wherein said mammal is a human being.

4. The method as defined in claim **1** wherein said IL-1β gene haplotype further comprises a thymidine nucleotide at position -511.

5. The method as defined in claim **1** wherein said inflammatory disorder is selected from the group consisting of coronary artery disease, osteoporosis, nephropathy in diabetes mellitus, alopecia areata, Graves' disease, systemic lupus erythematosus, lichen sclerosis, ulcerative colitis, periodontal disease, juvenile chronic arthritis, chronic iridocyclitis, psoriases, insulin dependent diabetes, diabetic complications, diabetic retinopathy, atherosclerosis, Crohn's disease, rheumatoid arthritis, osteoarthritis, congestive heart failure, and a neurodegenerative disease.

6. A method for detecting a mammalian patient who requires an increased dosage of an agent that reduces the effect of IL-1β comprising detecting, in a sample comprising DNA taken from said mammal, the presence of at least one copy of an IL-1β gene haplotype comprising cytosine nucleotides at positions -31 and +3953 in the patient, wherein the presence of at least one copy of the IL-1β gene haplotype indicates that the patient requires a dosage of the agent to reduce the effect of IL-1β in the patient that is higher than the dosage of the agent required to reduce the effect of IL-1β in a second mammal who does not have said at least one copy of the IL-1β haplotype.

7. The method as defined in claim **6** wherein said mammalian patient having at least one copy of the IL-1β gene haplotype has two copies of the IL-1β gene haplotype.

8. The method as defined in claim **6** wherein said mammalian patient is a human being.

9. The method as defined in claim **6** wherein said mammalian patient and said second mammal are of the same species.

10. The method as defined in claim **6** wherein said IL-1β gene haplotype further comprises a thymidine nucleotide at position -511.

11. The method as defined in claim **6** wherein said inflammatory disorder is selected from the group consisting of coronary artery disease, osteoporosis, nephropathy in diabetes mellitus, alopecia areata, Graves' disease, systemic lupus erythematosus, lichen sclerosis, ulcerative colitis, periodontal disease, juvenile chronic arthritis, chronic iridocyclitis, psoriases, insulin dependent diabetes, diabetic complications, diabetic retinopathy, atherosclerosis, Crohn's disease, rheumatoid arthritis, osteoarthritis, congestive heart failure, and a neurodegenerative disease.

12. A kit for detecting an increased risk of developing an inflammatory disorder in a mammal, comprising a first set of PCR primers for amplifying a first DNA sequence comprising the nucleotide at position -31 of an IL-1β gene, a second set of PCR primers for amplifying a second DNA sequence comprising the nucleotide at position +3953 of the IL-1β gene; and instructions for determining the presence of at least one copy of an IL-1β gene haplotype comprising cytosine nucleotides at positions -31 and +3953 in said mammal based on the size of the PCR products.

13. The kit as defined in claim **12** wherein said mammal having at least one copy the IL-1β gene haplotype comprising cytosine nucleotides at positions -31 and +3953 has two copies of the IL-1β gene haplotype.

14. The kit as defined in claim **12** wherein said mammal is a human being, and said inflammatory disorder is selected from the group consisting of coronary artery disease, osteoporosis, nephropathy in diabetes mellitus, alopecia areata, Graves' disease, systemic lupus erythematosus, lichen sclerosis, ulcerative colitis, periodontal disease, juvenile chronic arthritis, chronic iridocyclitis, psoriases, insulin dependent diabetes, diabetic complications, diabetic retinopathy, atherosclerosis, Crohn's disease, rheumatoid arthritis, osteoarthritis, congestive heart failure, and a neurodegenerative disease.

15. The kit as defined in claim **12** further comprising a third set of primers for amplifying a third DNA sequence comprising the nucleotide at position - 511 of the IL-1β gene and instructions for determining the presence of a copy of an IL-1β gene haplotype comprising cytosine nucleotides at positions -31 and +3953 and a thymidine nucleotide at position -511 in said mammal based on the size of the PCR products.


SEQUENCE LISTING

<110> Pfizer Products Inc.

<120> Methods and Reagents For Detecting Increased Risk Of Developing An
Inflammatory Disorder

<130> PC11026AJAK

<150> US 60/258034

<151> 2000-12-22

<160> 7

<170> PatentIn version 3.0

<210> 1

<211> 20

<212> DNA

<213> Homo sapiens

<400> 1
tggcattgat ctggttcatc 20

<210> 2

<211> 20

<212> DNA

<213> Homo sapiens

<400> 2
gtttaggaat cttcccactt 20

<210> 3

<211> 22

<212> DNA

<213> Homo sapiens

<400> 3
ccaatactct tttccccttt cc 22

<210> 4

<211> 20

<212> DNA

<213> Homo sapiens

<400> 4
cttgtgcctc gaagaggttt 20

<210> 5

<211> 19

<212> DNA

<213> Homo sapien

<400> 5
ttctccctcg ctgttttca 19

<210> 6

<211> 20

<212> DNA

<213> Homo sapien

<400> 6
gttgtcatca gactttgacc 20

<210>  7

<211>  20

<212>  DNA

<213>  HOMO SAPIEN

<400>  7
ttcagttcat atggaccaga                                                    20